Europäisches Patentamt

European Patent Office

Office européen des brevets

(11) Veröffentlichungsnummer: **0 214 342 B1**

# EUROPÄISCHE PATENTSCHRIFT

(45) Veröffentlichungstag der Patentschrift: **18.09.91**

(51) Int. Cl.⁵: **A61K 6/04, A61K 6/06**

(21) Anmeldenummer: **85115966.5**

(22) Anmeldetag: **13.12.85**

(54) **Werkstoff für verblendbaren Zahnersatz.**

(30) Priorität: **11.09.85 DE 3532329**

(43) Veröffentlichungstag der Anmeldung:
**18.03.87 Patentblatt 87/12**

(45) Bekanntmachung des Hinweises auf die
Patenterteilung:
**18.09.91 Patentblatt 91/38**

(84) Benannte Vertragsstaaten:
**AT BE CH DE FR GB IT LI LU NL SE**

(56) Entgegenhaltungen:
**EP-A- 0 055 383**
**EP-A- 0 115 058**
**DE-A- 3 135 034**
**DE-A- 3 405 218**

**PATENT ABSTRACTS OF JAPAN, Band 7, Nr.
86 (C-161)[1231], 9. April 1983; & JP-A-58 16
049 (TOKYO SHIBAURA DENKI K.K.)
29-01-1983**

(73) Patentinhaber: **Degussa Aktiengesellschaft
Weissfrauenstrasse 9
W-6000 Frankfurt am Main 1(DE)**

(72) Erfinder: **Groll, Werner, Dr. Dipl.-Ing.
Kardinal Döpfner Strasse 5
W-8757 Karlstein(DE)**
Erfinder: **Rothaut, Josef, Dr. Dipl.-Phys.
247 Harmon Avenue
Fort Lee, N.J. 07024(US)**

Rank Xerox (UK) Business Services

## Beschreibung

Die Erfindung betrifft einen Werkstoff für verblendbaren Zahnersatz mit einer metallischen Gefügematrix aus einem biokompatiblen Metall oder einer biokompatiblen Metallegierung.

Zur Herstellung von metallischem, verblendbarem Zahnersatz mit dem Wachsausschmelzverfahren werden hochedelmetallhaltige Legierungen verwendet, die gut verarbeitbar sind und eine hervorragende Biokompatibilität aufweisen. Diese Legierungen sind jedoch teuer, so daß in den letzten Jahren vermehrt preiswertere Legierungen mit verringerten Gold- und Platingehalten und zunehmenden Zusätzen an Palladium und Unedelmetallen entwickelt wurden. Diese Legierungen sind aber häufig schwer verarbeitbar, insbesondere bezüglich des Schmelz- und Gießverhaltens und der Lötbarkeit. Edelmetallfreie Legierungen werden ebenfalls für metallischen Zahnersatz verwendet, doch lassen sich solche Legierungen noch schwerer verarbeiten. Im Falle einer Keramikverblendung müssen die Legierungen in geeigneter Weise voroxidiert werden, um eine gute Haftung der Keramikverblendung auf der Legierung zu erzielen. Hierbei erhält man nicht immer eine für die Haftung optimale Oxidschicht, so daß sich oft Haftprobleme ergeben.

Aus der DE-OS 31 35 034 ist ein Werkstoff für Schmuck- und Gebrauchsgegenstände bekannt, der aus Edelmetall mit 1 bis 70 Vol.% Glas besteht, das eine Transformationstemperatur von 300 bis 500° C besitzen muß. Solche Werkstoffe sind allerdings als Zahnersatz nicht brauchbar, da es zu Verformungen während des Keramikbrandes kommt, die die Paßgenauigkeit des Zahnersatzes beeinträchtigt.

Die EP-A-0 115 058 beschreibt einen pulverförmigen Dentalwerkstoff auf der Grundlage von (a) Calciumaluminiumfluorosilicatglas, (b) für dentale Zwecke üblichen Edelmetallen, ihren Legierungen miteinander und/oder ihren Legierungen mit bis zu 40 % unedlen Metallen, bezogen auf das Gewicht der Legierung, und gegebenenfalls (c) trokkener Polycarbonsäure und/oder (d) chelatbildendem Mittel, dessen Herstellung und Verwendung.

Werkstoffe aus Metallen und Keramikteilchen sind in der Dentaltechnik als bindemittelhaltige Pasten bekannt, um auf dem Metallkern von Kronen und Brücken eine Zwischenschicht aufzubringen, die die Haftung einer äußeren Porzellanverblendung ermöglicht oder verbessert (z.B. EP-PS 55 383, DE-OS 30 27 473, DE-OS 25 25 274). Als Werkstoff für Kronen und Brücken sind diese Pasten aber nicht verwendet worden.

Es war daher Aufgabe der vorliegenden Erfindung, einen Werkstoff für verblendbaren Zahnersatz mit einer metallischen Gefügematrix aus einem biokompatiblen Metall oder einer biokompatiblen Metallegierung zu entwickeln, der preiswerter als hochedelmetallhaltige Legierungen ist, der sich problemlos verarbeiten läßt und der sich im Bedarfsfall sicher und ohne großen Aufwand direkt mit Keramik oder Kunststoff verblenden läßt, ohne Aufbringung einer Zischenschicht.

Diese Aufgabe wurde erfindungsgemäß dadurch gelöst, daß der Werkstoff der eingangs genannten Art neben dem metallischen Bestandteil 0,5 bis 30 Vol.% Keramikteilchen mit einer maximalen Korngröße von 40 µm enthält.

Vorzugsweise enthält der Werkstoff oxidische Teilchen, insbesondere oxidische Teilchen mit Schmelz- und Sintertemperaturen, die oberhalb der Schmelz- bzw. Sintertemperaturen der metallischen Komponenten liegen. Bewährt haben sich vor allem Werkstoffe, die 1 bis 12 Vol.% Keramikteilchen mit Teilchengröße $\leq$ 5 µm enthalten, wobei als Matrixbestandteil vorzugsweise Edelmetalle bzw. Edelmetallgemische verwendet werden. Allerdings kann die Metallmatrix auch Unedelmetalle enthalten bzw. aus Unedelmetall bestehen.

Die Herstellung dieser Werkstoffe erfolgt pulvermetallurgisch, indem Metallpulver mit einer maximalen Teilchengröße von 100 µm mit dem Keramikpulver innig gemischt, das Pulvergemisch verdichtet und anschließend gesintert wird. Preßdruck und Sintertemperatur richten sich nach den verwendeten Metallpulver. Es ist auch möglich, die Pulvermischung als Schlicker aufzubereiten und die geformte und verdichtete Schlickmasse nach dem Trocknen zu sintern.

Der Einbau von Keramikpulver in die metallische Gefügematrix bewirkt, daß man beim Modellieren des Zahnersatzes eine maximale Rohdichte erzielen kann, was die Schrumpfung beim Sintern des Modells minimiert und so die Paßgenauigkeit verbessert, ohne daß die Festigkeit sich verschlechtert.

In der Tabelle 1 sind für eine Legierung der Zusammensetzung Au50 Pt35 Pd15 die Auswirkungen der Zugabe verschiedener Keramikpulver (je 10 Vol.%) auf die Dichte und die 0,2 %-Dehngrenze nach und die lineare Schrumpfung beim Sintern zusammengestellt. Man erkennt, daß die Schrumpfung trotz zum Teil deutlich höherer Dichte nach dem Sintern kleiner ist als bei der keramikfreien Variante. Die 0,2%-Dehngrenze, erhöht sich bis auf eine Ausnahme ebenfalls durch den Keramikzusatz.

In Tabelle 2 ist für verschiedene Pulvergrößen und für verschiedene Legierungszusammensetzungen der Einfluß von $TiO_2$ auf die lineare Schrumpfung beim und die Dichte nach dem Sintern dargestellt. Auch hier wird eine erhöhte Dichte und 0,2%-Dehngrenze des gesinterten Werkstoffs bei Anwesenheit von $TiO_2$ beobachtet, während die Schrumpfung meist kleiner als bei der entsprechenden keramikfreien Variante ist.

Diese Beispiele zeigen, daß durch die Anwesenheit von Keramikpulvern in der metallischen Gefügematrix eine Erhöhung der Rohdichte erreicht werden kann.

Zur Herstellung von Zahnersatz wurde beispielsweise ein Werkstoff verwendet, bestehend aus 90 Vol.% eines Metallpulvergemisches aus 74,4 Gew.% Goldpulver $\leq$ 90 $\mu$m, 18,6 Gew.% Goldpulver $\leq$ 10 $\mu$m und 7 Gew.% Platin $\leq$ 15 $\mu$m mit 10 Vol.% Titandioxid, der bei 1200° C gesintert wurde. Verwendbar sind z.B. aber auch folgende Werkstoffe. 50 % Gold, 35 % Platin, 15 % Palladium; 50 % Gold, 35 % Platin, 10 % Palladium, 5 % Silber; oder 95 % Gold, 3 % Indium, 1 % Zinn.

## Tabelle 1

| Metall | Keramik 10 Vol.% | Pulvergröße μm | lineare Schrumpfung beim Sintern 1200°, Luft % | 0,2% Dehngrenze MPa | relative Dichte gesinterten Werkstoffs % |
|---|---|---|---|---|---|
| 50 % Gold 35 % Platin 15 % Palladium | – | $\leq$ 25 | 21,3 | 582 | 91,8 |
| " | $Bi_2O_3$ | < 3 | 21,3 | 610 | 92,3 |
| " | $SnO_2$ | < 1 | 17,5 | 415 | 91,6 |
| " | $ZrO_2$ | < 20 | 18,8 | 579 | 95,0 |
| " | $TiO_2$ | < 5 | 20,0 | 650 | 92,5 |
| " | $MgO$ | < 10 | 18,8 | 590 | 93,2 |

Tabelle 2

| Zusammensetzung | | | | | | | | | | | Eigenschaften | | |
|---|---|---|---|---|---|---|---|---|---|---|---|---|---|
| Gold | | Platin | | Palladium | | Silber | | Zinn + Iridium | | Titandioxid < 20 μm | lineare Schwindung | rel. Dichte gesint. Leg. | 0,2 % Dehngrenze |
| Konzentr. Gew.% | Teilchengröße μm | Konz. Gew.% | Teilchgr. μm | Konz. Gew.% | Teilchgr. μm | Konz. Gew.% | Teilchgr. μm | Konz. Gew.% | Teilchgr. μm | Vol.% | % | % | MPa |
| 50 | ≤ 25 | 35 | ≤ 15 | 15 | ≤ 15 | - | - | - | - | 0 | 21,3 | 91,8 | 582 |
| 50 | ≤ 25 | 35 | ≤ 15 | 15 | ≤ 15 | - | - | - | - | 10 | 20,0 | 92,0 | 610 |
| 50 | ≤ 25 | 35 | ≤ 15 | 15 | ≤ 15 | - | - | - | - | 20 | 18,8 | 91,9 | 650 |
| 50 | ≤ 50 | 35 | ≤ 15 | 15 | ≤ 15 | - | - | - | - | 0 | 20,0 | 91,8 | 630 |
| 50 | ≤ 50 | 35 | ≤ 15 | 15 | ≤ 15 | - | - | - | - | 10 | 20,0 | 97,2 | 692 |
| 50 | ≤ 100 | 35 | ≤ 15 | 15 | ≤ 15 | - | - | - | - | 0 | 20,0 | 92,3 | 625 |
| 50 | ≤ 100 | 35 | ≤ 15 | 15 | ≤ 15 | - | - | - | - | 10 | 18,8 | 93,3 | 620 |
| 50 | ≤ 50 | 35 | ≤ 15 | 10 | ≤ 15 | 5 | ≤ 15 | - | - | 0 | 17,5 | 84,8 | 550 |
| 50 | ≤ 50 | 35 | ≤ 15 | 10 | ≤ 15 | 5 | ≤ 15 | - | - | 10 | 17,5 | 90,0 | 580 |
| 95,5 | ≤ 50 | - | - | - | - | - | - | 4,5 | ≤ 25 | 0 | 17,6 | 84,7 | 60 |
| 95,5 | ≤ 50 | - | - | - | - | - | - | 4,5 | ≤ 25 | 2 | 17,0 | 89,4 | 85 |
| 95,5 | ≤ 50 | - | - | - | - | - | - | 4,5 | ≤ 25 | 4 | 17,0 | 91,1 | 95 |

## Patentansprüche

1. Werkstoff für verblendbaren Zahnersatz mit einer metallischen Gefügematrix aus einem biokompatiblen Metall bzw. einer biokompatiblen Metallegierung,

4

dadurch gekennzeichnet,
daß er neben dem metallischen Bestandteil 0,5 bis 30 Vol. % Keramikteilchen mit einer maximalen Korngröße von 40 μm enthält.

2.  Werkstoff nach Anspruch 1,
dadurch gekennzeichnet,
daß er oxidische Teilchen enthält.

3.  Werkstoff nach Anspruch 1 oder 2,
dadurch gekennzeichnet,
daß die Schmelz- und Sintertemperaturen der Keramikteilchen oberhalb der Schmelz- und Sintertemperaturen der metallischen Komponenten liegen.

4.  Werkstoff nach Anspruch 1 bis 3,
dadurch gekennzeichnet,
daß er 1 bis 12 Vol. % Keramikteilchen mit einer maximalen Teilchengröße von 5 μm enthält.

5.  Werkstoff nach Anspruch 1 bis 4,
dadurch gekennzeichnet,
daß die Metallmatrix aus Edelmetall besteht.

## Claims

1.  A material for faceable false teeth comprising a metallic structural matrix of a biocompatible metal or a biocompatible metal alloy, characterized in that, in addition to the metallic component, it contains 0.5 to 30% by volume ceramic particles having a maximum particle size of 40 μm.

2.  A material as claimed in claim 1, characterized in that it contains oxidic particles.

3.  A material as claimed in claims 1 or 2, characterized in that the melting and sintering temperatures of the ceramic particles are above the melting and sintering temperatures of the metallic components.

4.  A material as claimed in claims 1 to 3, characterized in that it contains 1 to 12% by volume ceramic particles having a maximum particle size of 5 μm.

5.  A material as claimed in claims 1 to 4, characterized in that the metal matrix consists of noble metal.

## Revendications

1.  Matériau pour prothèse dentaire apte au revêtement, comportant une matière de base structurale métallique en un métal biocompatible ou en un alliage de métaux biocompatible, caractérisé en ce qu'il contient, outre le composant métallique, 0,5 à 30 % en volume de particules céramiques ayant une taille maximale de particule de 40 μm.

2.  Matériau selon la revendication 1, caractérisé en ce qu'il contient des particules d'oxyde.

3.  Matériau selon la revendication 1 ou 2, caractérisé en ce que les températures de fusion et de frittage des particules céramiques sont supérieures aux températures de fusion et de frittage des composants métalliques.

4.  Matériau selon l'une quelconque des revendications 1 à 3, caractérisé en ce qu'il contient de 1 à 12 % en volume de particules céramiques ayant une taille maximale de particule de 5 μm.

5.  Matériau selon l'une quelconque des revendications 1 a 4, caractérisé en ce que la matière de base métallique est constitué de métal noble.